# EUROPÄISCHE PATENTSCHRIFT

(11) **EP 1 307 474 B1**
(45) Veröffentlichungstag und Bekanntmachung des Hinweises auf die Patenterteilung: **03.03.2010**
(21) Anmeldenummer: 01960574.0
(22) Anmeldetag: 27.07.2001
(51) Int. Cl.: C07K 14/215

(54) **PROTONEN-TRANSLOZIERENDE RETINALPROTEINE MIT KONSTANTEM ALL-TRANS-RETINAL-GEHALT UND VERLANGSAMTEN PHOTOZYKLUS**
PROTON-TRANSLOCATING RETINAL PROTEIN
PROTEINE CONTENANT DU RETINAL TRANSLOCATRICE DE PROTONS

(30) Priorität: 27.07.2000 DE 10036745; 29.09.2000 DE 10048383
(43) Veröffentlichungstag der Anmeldung: 07.05.2003
(73) Patentinhaber: MIB - Munich Innovative Biomaterials GmbH, 82152 Planegg-Martinsried (DE)
(72) Erfinder: OESTERHELT, Dieter, 81377 München (DE); HAMPP, Norbert, 35287 Amöneburg-Rossdorf (DE); PFEIFFER, Matthias, 35037 Marburg (DE)
(74) Vertreter: Bremi, Tobias Hans
(86) Internationale Anmeldenummer: PCT/EP2001/008715
(87) Internationale Veröffentlichungsnummer: WO 2002/010207

(56) Entgegenhaltungen:
- WO-A-94/05008
- DELANEY JOHN K ET AL: "The residues Leu 93 and Asp 96 act independently in the bacteriorhodopsin photocycle: Studies with the Leu 93 ->Ala, Asp 96 -> Asn double mutant." BIOPHYSICAL JOURNAL, Bd. 70, Nr. 5, 1996, Seiten 2366-2372, XP001026895 ISSN: 0006-3495
- OESTERHELT D ET AL: "BACTERIORHODOPSIN A BIOLOGICAL MATERIAL FOR INFORMATION PROCESSING" QUARTERLY REVIEWS OF BIOPHYSICS, Bd. 24, Nr. 4, 1991, Seiten 425-478, XP002119796 ISSN: 0033-5835

## Beschreibung

Die Erfindung betrifft ein Protonen-translozierendes Retinalprotein, eine das Protonen-translozierende Retinalprotein enthaltende photochrome Zusammensetzung und die Verwendung des Protonen-translozierendes Retinalproteins und der Zusammensetzung.

Halobakterien sind Archaea, die neben den Bacteria und Eukarya eine dritte Domäne des Lebens bilden. Archaea verdanken ihren Namen den ungewöhnlichen Biotopen, in denen sie vorkommen. Sie leben häufig unter "archaischen" Bedingungen, d.h. bei extremen Temperaturen, Salzkonzentrationen oder pH-Werten, wie sie auf der frühen Erdoberfläche geherrscht haben könnten.

Die halophilen Archaea umfassen 9 Genera (z.B. *Halobacterium, Haloferax, Natronomonas,* etc.) und sind durchwegs extremophil, d.h. sie leben in Salzlösungen, deren Konzentration von 2 molar bis zur Sättigung reicht und die manchmal noch zusätzlich alkalische pH-Werte bis zu pH 11 aufweisen. In der Natur sind die Halobakterien Teil eines komplexen Ökosystems. Die bei permanenter Sonneneinstrahlung wachsende Salinität in Salzgewinnungsanlagen oder die Bedingungen im Toten Meer und anderen natürlichen hypersalinen Gewässern erlauben nach den jährlichen Regengüssen zunächst das photolitotrophe Wachstum der halotoleranten Grünalge *Duniella parva* bis zu einem Salzgehalt von etwa 12%. Nach Überschreiten der für sie optimalen Bedingungen stirbt *Duniella* ab und ermöglicht das massive Wachstum von halophilen Archaea, die auf Grund ihres Carotinoidgehalts oft zu einer Rotfärbung dieser Gewässer führen.

Die halophilen Archaea besitzen neben Fermentation, aerober und anaerober Atmung eine zusätzliche Möglichkeit der Energieumwandlung, die unter den Archaea einmalig ist: sie können mittels Retinal-abhängiger Photosynthese Energie aufnehmen und umwandeln. Im Gegensatz zu der grünen, Chlorophyll-abhängigen Photosynthese ist hier nur ein einziges Protein an der Energieaufnahme und -umwandlung beteiligt, nämlich ein lichtgetriebenes Protonen-translozierendes Retinalprotein.

Das bekannteste Beispiel eines solchen Protonen-translozierenden Retinalproteins ist die archaebakterielle Protonenpumpe Bakteriorhodopsin (BR), die die Lichtenergie direkt zur Erzeugung eines elektrochemischen Protonengradienten, der in chemische Energie gewandelt wird, ausnutzt. Bakteriorhodopsin ist ein intrinsisches Membranprotein mit einem Molekulargewicht von ca. 26 kDa. Die Polypeptidkette durchquert die Membran siebenmal und bildet so eine Sekundärstruktur von sieben helikalen Transmembranbereichen. An die Seitenkette eines Lysins der siebenten Helix im Inneren des Proteins ist kovalent ein Retinal (Vitamin A Aldehyd) gebunden. Die entstandene CH=N-Gruppe wird Schiff'sche Base (SB) genannt und ist im Ausgangszustand am Stickstoff protoniert (SBH). Einen Überblick über das Bakteriorhodopsin gibt die Arbeit von Haupts et al. (Annu. Rev. Biophys. Biomol. Struct. 28, 367-99, 1999).

Der Chromophor des BR absorbiert gelbgrünes Licht maximal bei 570 nm, so dass BR dem menschlichen Auge violett erscheint. Nach Absorption eines Photons erfährt das Protein chemische und strukturelle Änderungen, die zu unterscheidbaren, spektroskopisch messbaren Intermediaten führen. Sie werden mit den Buchstaben K, L, M, N und O bezeichnet und jeweils mit der Wellenlänge der maximalen Absorption indiziert. Der Zyklus kann vereinfacht folgendermaßen beschrieben werden:

BR₅₇₀→ K₅₉₀→ L₅₅₀→ M₄₁₀→ N₅₆₀→ O₆₄₀→ BR₅₇₀

Bakteriorhodopsin ist das bisher einzige Retinalprotein, das in der Natur in Form eines zweidimensionalen Kristalles vorkommt. Im Bakterium sind die Kristalle in der sogenannten Purpurmembran lokalisiert. Die Organisation in der Purpurmembran stabilisiert das Protein in einem solchen Maße, dass es für eine Reihe technischer Anwendungen vorgeschlagen worden ist (zusammengefaßt in Oesterhelt et al., Quarterly Rev. Biophysics 24, 425-478, 1991). Dabei können die bei Belichtung auftretenden Änderungen des pH-Wertes der Lösung, der elektrischen Spannung und der Farbe genutzt werden.

So ist der Farbwechsel vom Violett des Bakteriorhodopsins im Ausgangszustand BR₅₇₀ zum Gelb im Intermediat M₄₁₀ die Basis für Anwendungen in der optischen Informationstechnologie. Der Zerfall des Intermediates M₄₁₀, das eine Lebensdauer von wenigen Millisekunden hat, ist der geschwindigkeitsbestimmende Schritt in diesem Zyklus. Die Lichtintensität und die thermische Zerfallskonstante des M-Intermediats bestimmen im Photozyklus das Verhältnis der Farben Violett und Gelb.

Der Retinylidenteil des Bakteriorhodopsins liegt im Dunkeln als eine Mischung von all-*trans*, 15-*anti* und 13-*cis*, 15-*syn* Konfiguration im Verhältnis von etwa 60:40 vor. Es ist ausschließlich die all-*trans* Form des Chromophors, die den physiologischen Prozeß der Protonentranslokation vermittelt und das gelbe Intermediat M₄₁₀ durchläuft ("*trans*-Zyklus"). Zwar führt auch die Absorption von Photonen in der 13-*cis* Konfiguration zu einem Zyklus von Farbänderungen, dem "*cis*-Zyklus", dieser unterscheidet sich jedoch vom *trans-*Zyklus" darin, dass kein gelbes M-ähnliches Intermediat gebildet wird. Aus dem "*cis*-Zyklus" springt das Molekül bei Belichtung mit einer sehr kleinen Wahrscheinlichkeit in den *trans*-Zyklus" hinüber. Dieser Wechsel vom *"cis-"* in den "*trans*-Zyklus" wird als Helladaptation der Dunkel-adaptierten Form bezeichnet. In der Praxis bedeutet das, dass nach Dunkelaufbewahrung einer Probe (Dunkeladaptation) eine anfängliche Belichtung nur zu etwa 60% des theoretisch möglichen M-Intermediats führt. Durch weitere Belichtung adaptiert die Probe nach und nach (Helladaptation), so dass schließlich alle Moleküle in den "*trans*-Zyklus" überführt sind und das M-Intermediat durchlaufen.

Der Wechsel der Moleküle vom *"cis-"* in den "*trans*-Zyklus" führt zu einer Verschiebung des Absorptionsmaximums um mehrere nm und bedeutet einen erheblichen Nachteil für die Verwendung von Bakteriorhodopsinen bei der Herstellung photochromer Erzeugnisse, z.B. optischer Filme oder Druckfarben.

Es wäre deshalb wünschenswert, Protonen-translozierende Retinalproteine herzustellen, deren Absorptionsmaximum im Dunkel-adaptierten Zustand dem im Hell-adaptierten Zustand möglichst genau entspricht. Darüberhinaus wäre es von Vorteil, wenn die Stabilität des M-Intermediates erhöht werden könnte, um den Farbwechsel der im "*trans*-Zyklus" vorliegenden Moleküle von Violett nach Gelb möglichst genau beobachten zu können.

Erfindungsgemäß wird nunmehr ein Protonen-translozierendes Retinalprotein bereitgestellt, das aus der Gruppe von:
(i) Muteinen eines natürlichen Protonen-translozierenden Retinalproteins aus halophilen Archaebakterien, die einen verlangsamten Photozyklus aufweisen (Typ 1-Mutation) und deren all-*trans* Retinal-Gehalt im Hell- und Dunkel-adaptierten Zustand um nicht mehr als 10% voneinander abweicht (Typ 2-Mutation) und/oder
(ii) Homologen der Muteine (i) mit verlangsamten Photozyklus, deren Retinalisomeren-Zusammensetzung im Hell- und Dunkel-adaptierten Zustand um nicht mehr als 10% voneinander abweicht
ausgewählt ist.

Unter einem Mutein werden durch eine Substitution, Deletion oder Insertion modifizierte Protonen-translozierende Retinalproteine verstanden. Muteine können ein oder mehrere Mutationen aufweisen. Typ 1-Mutationen sind dabei Mutationen, die zu einer Verlangsamung des Photozyklus im Vergleich zum natürlichen Retinalprotein aus *Halobacterium salinarum* (SEQ ID No.1) führen. Die Messung des Photozyklus erfolgt dabei wie z.B. von Miller & Oesterhelt, Biochem. Biophys. Acta 1020, 57-64, 1990, beschrieben. Typ 2-Mutationen führen zu einer im Vergleich zum natürlichen Retinalprotein aus *Halobacterium salinarum* (SEQ ID No.1) erhöhten Konstanz des all-*trans*-Retinal-Gehalts im Hell- und im Dunkel-adaptierten Protein. Der all-*trans*-Retinal-Gehalt wird dabei bestimmt, wie von Tittor et al., Biophys. J. 67,'1682-1690, 1994, beschrieben. Die Prozentangabe bezieht sich auf den Gesamtgehalt der mittels des erwähnten Verfahrens bestimmbaren Retinalisomere. Normalerweise werden Typ 1-Mutationen und Typ 2-Mutationen verschiedene Aminosäuren betreffen. Es ist jedoch auch möglich, dass eine einzelne Mutation beide gewünschten Wirkungen aufweist und daher gleichzeitig als Typ 1- und als Typ 2-Mutation zu klassifizieren ist. Soweit natürlich auftretende, archaebakterielle Protonen-translozierende Retinalproteine im Vergleich zu dem bekanntesten Protonen-translozierenden Retinalprotein, d.h., Bakteriorhodopsin aus *Halobacterium salinarum* (SEQ ID No.1), einen verlangsamten Photozyklus aufweisen und ihr all-*trans*-Retinal-Gehalt im Hell- und im Dunkel-adaptierten Zustand um nicht mehr als 10% voneinander abweicht, gelten sie im Rahmen der vorliegenden Erfindung ebenfalls als Muteine.

Der dem Fachmann bekannte Ausdruck "Homolog" bezeichnet eine Verwandtschaft zwischen zwei oder mehr Peptiden, Polypeptiden oder Proteinen, der durch die Obereinstimmung zwischen den Sequenzen mittels bekannter Verfahren, z. B. der computergestützten Sequenzvergleiche (Basic local alignment search tool, S.F. Altschul et al., J. Mol. Biol. 215 (1990), 403-410), bestimmt werden kann. Der Prozentsatz der Identität ergibt sich aus dem Prozentsatz identischer Bereiche in zwei oder mehr Sequenzen unter Berücksichtigung von Lücken oder anderen Sequenzbesonderheiten. In der Regel werden spezielle Computerprogramme mit Algorithmen eingesetzt, die den besonderen Anforderungen Rechnung tragen.

Bevorzugte Verfahren zur Bestimmung der Homologie erzeugen zunächst die größte Übereinstimmung zwischen den untersuchten Sequenzen. Computerprogramme zur Bestimmung der Identität zwischen zwei Sequenzen umfassen, sind jedoch nicht eingeschränkt auf das GCG Programmpaket, einschließlich GAP (Devereux, J., et al., Nucleic Acids Research 12 (12):387 (1984); Genetics Computer Group University of Wisconsin, Madison, (WI)); BLASTP, BLASTN, und FASTA (Altschul, S. et al., J. Mol. Biol. 215:403-410) (1999)). Das BLASTX Programm kann vom National Centre for Biotechnology Information (NCBI) und aus weiteren Quellen bezogen werden (BLAST Handbuch, Altschul S., et al., NCB NLM NIH Bethesda MD 20894; Altschul, S., et al., Mol. Biol. 215:403-410 (1990)). Auch der bekannte Smith Waterman-Algorithmus kann zur Bestimmung des Prozentsatzes der Identität verwendet werden.

Bevorzugte Parameter für den Aminosäuresequenz-Vergleich umfassen die nachstehenden:

| | |
|---|---|
| Algorithmus: | Needleman und Wunsch, J. Mol. Biol 48:443-453 (1970) |
| Vergleichsmatrix: | BLOSUM 62 aus Henikoff und Henikoff, PNAS USA 89 (1992), 10915-10919 |
| Lücken-Wert (Gap Penalty): | 12 |
| Lückenlängen-Wert: (Gap Length Penalty): | 4 |
| Homologie-Schwellenwert: (Threshold of Similarity): | 0 |

Das GAP-Programm ist auch zur Verwendung mit den vorstehenden Parametern geeignet. Die vorstehenden Parameter sind die Fehler-Parameter (default parameters) für Aminosäuresequenz-Vergleiche, wobei Lücken an den Enden den Wert nicht verändern. Die Erfindung umfasst daher auch Fusionsproteine, d.h. Protonen-translozierende Retinalproteine mit einem Fusionsproteinanteil. Bei sehr kurzen Sequenzen im Vergleich zur Referenz-Sequenz kann es weiterhin notwendig sein, den Erwartungswert auf bis zu 100.000 (expectation value) zu erhöhen und gegebenenfalls die Wortlänge (word size) auf bis zu 2 zu verkleinern.

Weitere beispielhafte Algorithmen, Lücken-Öffnungs-Werte (gap opening penalties), Lükkenausdehnungs-Werte (gap extension penalties), Vergleichsmatrizen einschließlich der im Programm-Handbuch, Wisconsin-Paket, Version 9, September 1997, genannten können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Eine mit dem oben genannten Algorithmus ermittelte Übereinstimmung von 40% wird im Rahmen dieser Anmeldung als 40% Identität bezeichnet. Entsprechendes gilt für höhere Prozentsätze.

Überraschenderweise hat sich nunmehr gezeigt, dass die Kombination einer Typ 1-Mutation und einer Typ 2-Mutation im erfindungsgemäßen Protonen-translozierenden Retinalprotein zu einer weitgehenden Konstanz des all-*trans*-Retinal-Gehaltes sowie zu einer Verlangsamung des Photozyklus führen kann. Der all-*trans*-Retinal-Gehalt der Dunkel-adaptierten Form des erfindungsgemäßen Protonen-translozierenden Retinalproteins unterscheidet sich von dem der Hell-adaptierten Form in der Regel um nicht mehr als 10%. In bevorzugten Ausführungsformen sind die Unterschiede sogar geringer und liegen bei maximal 8 oder sogar maximal 5%. Überraschenderweise hat sich weiterhin gezeigt, dass gerade Muteine, deren all-*trans*-Retinal-Gehalt sich im Hell- und im Dunkel-adaptierten Zustand nicht wesentlich, d.h., um nicht mehr als 10% unterscheidet, einen all-*trans*-Retinal-Gehalt von mindestens 60% in Hell- wie im Dunkel-adaptierten Zustand aufweisen. Dieser unerwartete Nebeneffekt ist äußerst vorteilhaft, da jede Erhöhung des Anteils der Moleküle, die am "*trans*-Zyklus" teilnehmen, bei Belichtung zu einem deutlicheren Farbwechsel und damit zu einer Verbesserung der optischen Eigenschaften führt.

Erfindungsgemäß werden Muteine bereitgestellt, die einen konstanten all-*trans*-Retinal-Gehalt im Bereich von mindestens 60 bis 100%, bevorzugt 62% oder 65% bis 100% aufweisen. Während die meisten Retinalproteine einen all-*trans*-Retinal-Gehalt im Bereich von 60 oder 65% bis 85% aufweisen, sehen besonders bevorzugte Ausführungsformen einen all-*trans*-Retinal-Gehalt von mindestens 70% oder 75%, am besten sogar 80% bis 100% vor.

In einer bevorzugten Ausführungsform ist das natürliche Protonen-translozierende Retinalprotein, dessen Mutein(e) die oben erwähnten Eigenschaften aufweisen, ein archaebakterielles Bakteriorhodopsin, z.B. ein halobakterielles Rhodopsin, bevorzugt Bakteriorhodopsin (SEQ ID No.1) aus *Halobacterium salinarum.*

Das erfindungsgemäße Protonen-translozierende Retinalprotein umfasst Muteine mit einer Typ 1- und einer Typ 2-Mutation, deren Aminosäuresequenz mit der Aminosäuresequenz SEQ ID No.1 eine Identität von mindestens 40% aufweist. In weiteren Ausführungsformen beträgt die Identität mindestens 50, 60 oder 70%. In besonders bevorzugten Ausführungsformen beträgt die Identität mindestens 80, 90 oder 95%.

In einer weiteren bevorzugten Ausführungsform handelt es sich bei dem Protonen-translozierenden Retinalprotein um ein Homolog mit einer Aminosäuresequenz, das im Bereich der C-Helix und/oder der F-Helix eine Identität von mindestens 60% mit den entsprechenden Aminosäuresequenzen des reifen Bakteriorhodopsins aus SEQ ID No.1 aufweist. In weiter bevorzugten Ausführungsformen liegt der Prozentsatz der Identität für die C-Helix und/oder die F-Helix bei mindestens 70, 80 oder 90%, bevorzugt bei mindestens 95%. Der Identitätsgrad für die C- und die F-Helix kann dabei gleich oder verschieden sein.

Der Photozyklus des erfindungsgemäßen Protonen-translozierenden Retinalproteins wird durch die Typ 1-Mutation verlangsamt. Die thermische Zykluszeit beträgt in jedem Fall mehr als 10 ms, bevorzugt mehr als 1 s oder gar mehr als 10 s. In besonders bevorzugten Ausführungsformen liegt die thermische Zykluszeit im Minutenbereich, d.h. sie beträgt mehr als 1 min, in weiteren bevorzugten Ausführungsformen sogar mehr als 5 oder 10 min. Die thermische Zykluszeit kann im Extremfall bis zu 2 Stunden betragen, in der Regel jedoch nicht mehr als 90 oder 60 min.

Die Typ 1-Mutation des Protonen-translozierenden Retinalproteins kann in einem Aminosäureaustausch an einer oder mehreren der Aminosäurepositionen bestehen, die im natürlichen Protein am katalytischen Zyklus beteiligt sind. Die vorliegende Erfindung umfaßt daher Retinalproteine, bei denen z.B. eine oder mehrere der Positionen der an der Protonen-Translokation beteiligten Aminosäuren aus der Gruppe der Aminosäurereste D38, R82, D85, D96, D102, D104, E194 und/oder E204 gemäß SEQ ID No.1 bzw. der ihnen entsprechenden Aminosäurereste in homologen Proteinen verändert ist. Ein bevorzugter Aminosäureaustausch ist D96N, d.h. die Aminosäure D an Position 96 von SEQ ID No.1 bzw. die entsprechenden Aminosäure in einem homologen Protein wird durch N ersetzt. Weiter bevorzugte Aminosäureaustausche sind D38R und D102R und/oder D104R.

Die Typ 2-Mutation der Retinalproteine der erfindungsgemäßen Retinalproteine führt zu einem Aminosäureaustausch an einer oder mehrerer der Aminosäurepositionen, die die Retinalbindungstasche bilden, und/oder unmittelbar benachbarter Positionen. Unter der Retinalbindungstasche wird die Summe der Aminosäuren verstanden, die der Schiff'schen Base des Retinals ihre charakteristischen chemischen und physikalischen Eigenschaften verleihen. Die die Retinalbindungstasche bildenden Aminosäuren bzw. unmittelbar benachbarte Aminosäuren sind ausgewählt aus der Gruppe der Aminosäurereste Val49, Ala53, L93, Met118, Gly122, S141 und Met145 gemäß SEQ ID No.1 bzw. der ihnen entsprechenden Aminosäurereste in homologen Proteinen. In bevorzugten Ausführungsformen ist die Typ 2-Mutation V49A, V49G, V49F, L93A, G122K, G122C, G122M, S141A, S141M, M1451, M145F, M145W, M145C oder M145K. Diese Typ 2-Mutationen bewirken überraschenderweise eine Konstanz des all-*trans* Anteils im Retinylidenteil des Bakteriorhodopsins im Hell- und im Dunkel-adaptierten Zustand.

Absorptionsmaxima und Retinalisomerenverhältnisse des *Halobacterium salinarum* Wildtyps (WT), der reinen Typ 1-Mutante D96N, der reinen Typ 2-Mutanten M145F und L93A sowie der erfindungsgemäßen Retinalproteine D96N-M145F und D96N-L93A sind in der nachfolgenden Tabelle gezeigt:

| **Stamm** | **λₘₐₓ in nm** | | **Isomere** | | | |
|---|---|---|---|---|---|---|
| | **DA** | **HA** | **DA** | | **HA** | |
| | | | **all*-trans*** | **13-*cis*** | **all-*trans*** | **13-*cis*** |
| WT | 560 | 568 | 60 | 40 | 98 | 2 |
| D96N | 560 | 569 | 54 | 46 | 96 | 4 |
| M145F | 558 | 559 | 72 | 28 | 86 | 14 |
| D96N-M145F | 560 | 560 | 66 | 34 | 67 | 33 |
| L93A | 541 | 541 | 80 | 20 | 82 | 18 |
| D96N-L93A | 544 | 544 | 80 | 20 | 81 | 19 |

| | | | | | | |
|---|---|---|---|---|---|---|
| Anm.: "DA" bedeutet "Dunkel-adaptiert" "HA" bedeutet "Hell-adaptiert" | | | | | | |

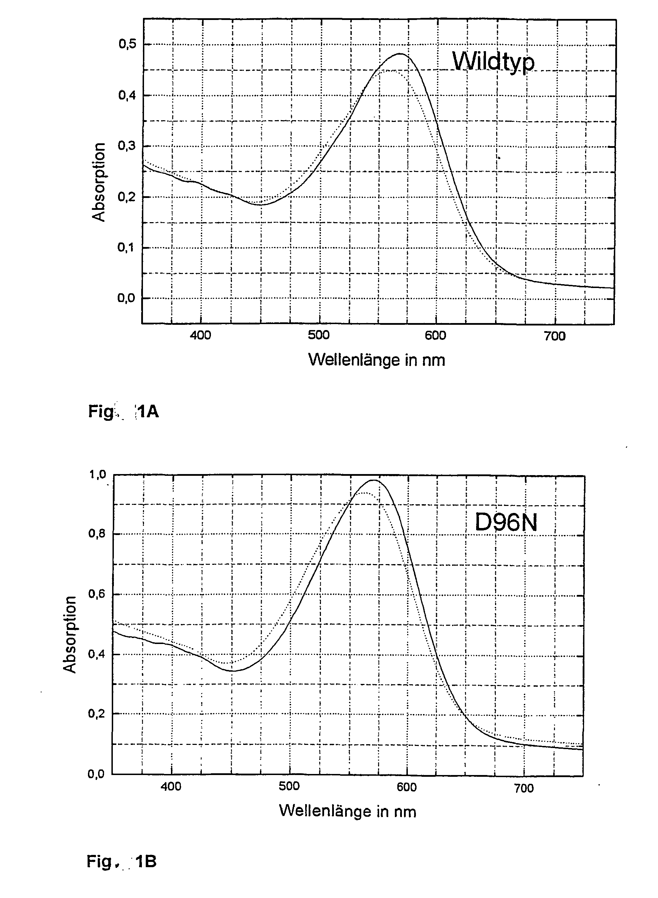

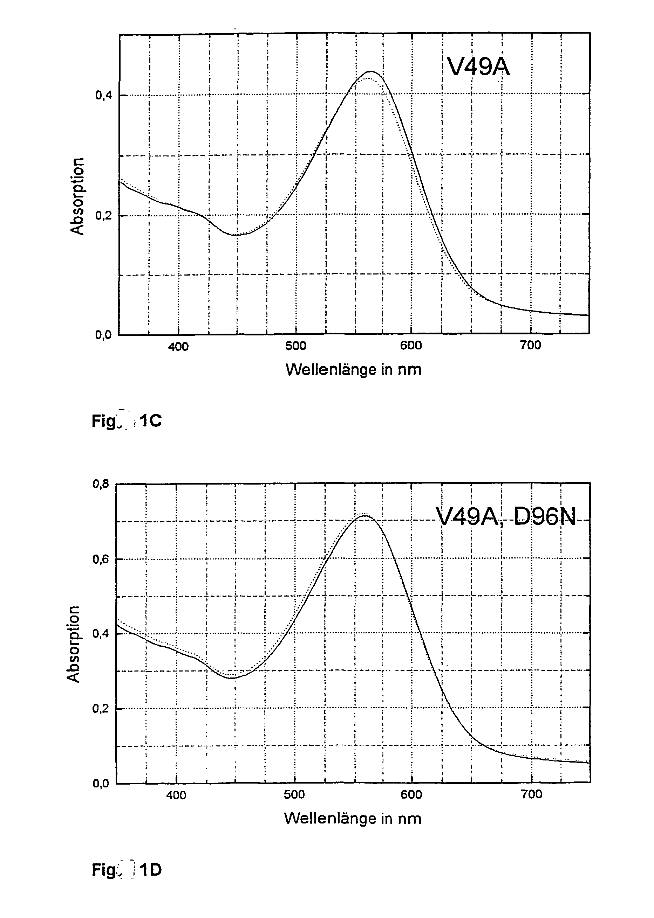

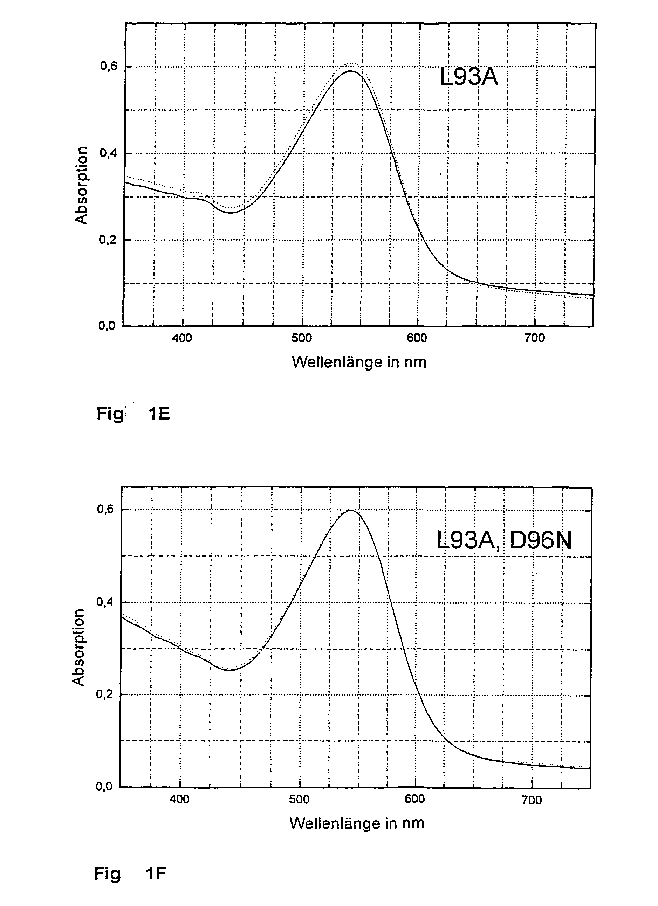

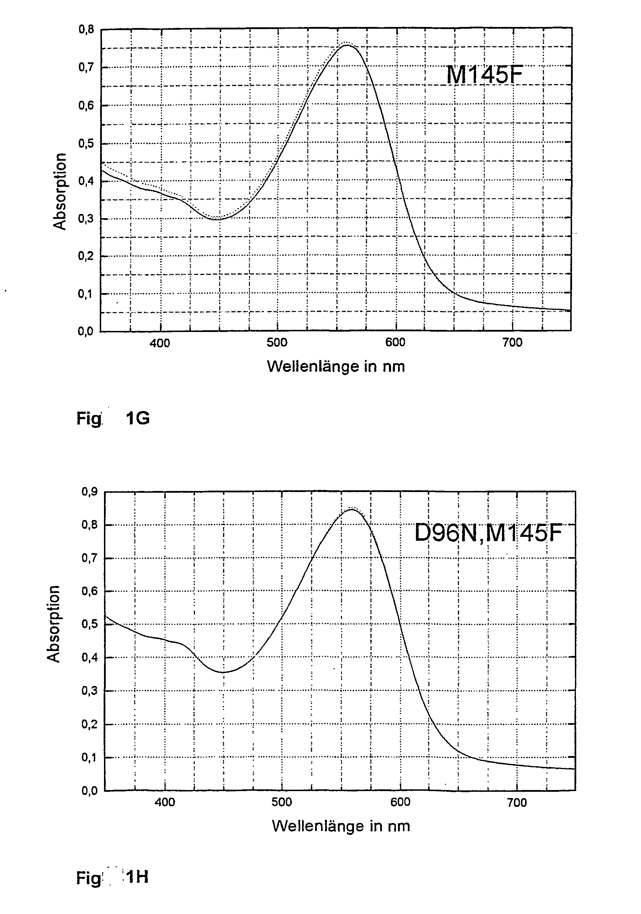

Besonders bevorzugte Kombinationen von Typ 1- und Typ 2-Mutationen sind beispielsweise V49A-D96N, L93A-D96N und M145F-D96N (s. Figur 1).

In einer weiteren Ausführungsform liegen die erfindungsgemäßen Retinalproteine in membrangebundener Form vor, wobei die Membran eine Dichte zwischen 1,10 und 1,20 g/cm³ aufweist. In einer bevorzugten Ausführungsform beträgt die Dichte zwischen 1,175 und 1,185 g/cm³. Besonders bevorzugt ist die Form einer Purpurmembran mit einer Dichte von 1,18 g/cm³_{.}

Erfindungsgemäß werden weiterhin Nukleinsäuren bereitgestellt, die für die oben beschriebenen Retinalproteine kodieren. Diese Nukleinsäuren können einmal durch Mutationen bereits bekannter Gene für Protonen-translozierende Retinalproteine, z.B. des für das Bakteriorhodopsin aus *Halobacterium salinarum* kodierenden Genes erzeugt werden (Dunn et al., Proc. Natl. Acad. Sci. USA 78, 6744-6748, 1981), zum anderen nach bekannten Verfahren vollsynthetisch hergestellt werden. Da der genetische Code der Archaea sich von dem der Prokarya und Eukarya nicht unterscheidet, können auch zur Transformation in Halobakterien vorgesehene Nukleinsäuren nach den bekannten Regeln hergestellt werden. Der Fachmann wird sich allerdings bemühen, eine auf den jeweils vorgesehenen Wirtsorganismus abgestimmte Codon-Usage zu berücksichtigen. Bei den erfindungsgemäßen Nukleinsäuren kann es sich um Ribonukleinsäuren und/oder Desoxyribonukleinsäuren handeln.

Erfindungsgemäß werden weiterhin Vektoren zur Verfügung gestellt, die die für die Protonen-translozierenden Retinalproteine kodierenden Nukleinsäuren umfassen. Je nach dem für diesen Vektor vorgesehenen Wirtsorganismus wird der Fachmann zwischen archaebakteriellen Vektoren, Vektoren für die Expression in Prokarya (*E. coli, Bacillus, Pseudomonas, Klebsiella* etc.) oder Eukarya (Hefe, Tierzellkulturen (CHO, HeLa, COS etc.), Pflanzen oder Pflanzenzellen, Insektenzellen) auswählen.

Erfindungsgemäß wird weiterhin eine Wirtszelle bereitgestellt, die eine für ein erfindungsgemäßes Protonen-translozierendes Retinalprotein kodierende Nukleinsäure oder einen erfindungsgemäßen Vektor enthält. Bei den Wirtszellen handelt es sich z.B. um Archaea, bevorzugt Halobakterien, besonders bevorzugt *Halobacterium salinarum,* dessen Transformation beschrieben ist (Cline et al., Can. J. Microbiol. 35, 148-152, 1989). Alternativ können erfindungsgemäße Vektoren auch in *E*. *coli* oder anderen prokaryontischen Wirten, bei Bedarf auch in den oben erwähnten eukaryontischen Zellen exprimiert werden.

Erfindungsgemäß wird weiterhin eine photochrome Zusammensetzung bereitgestellt, die zusätzlich zu einem erfindungsgemäßen Protonen-translozierenden Retinalprotein Stabilisatoren, die Schaumbildung vermindernde und/oder UV-Licht absorbierende Zusatzstoffe und/oder Puffersubstanzen enthalten kann.

Die erfindungsgemäße photochrome Zusammensetzung kann z.B. Glycerin, organische Polymere und/oder organische Lösungsmittel enthalten.

Die erfindungsgemäßen Protonen-translozierenden Retinalproteine oder die sie enthaltenen photochromen Zusammensetzungen können zum Herstellen von optischen Filmen verwendet werden. Die Herstellung optischer Filme aus Bakteriorhodopsinen ist bereits bekannt und z.B. in Hampp et al., SPIE 3623, 243, 1999 ausführlich beschrieben. Ein unter Verwendung der erfindungsgemäßen Protonen-translozierenden Retinalproteine hergestellter optischer Film ist für optische Aufzeichnungen geeignet. Eine weitere Verwendungmöglichkeit solcher optischen Filme ist mit der Interferometrie oder bei der holographischen Mustererkennung gegeben. Auch die Verwendung optischer Filme als optischer Lichtmodulator ist möglich. Darüberhinaus ist die Herstellung volumenhafter Speicher zur optischen Datenspeicherung denkbar (Birge, Scientific American, 1995, 66).

Die vorliegende Erfindung stellt des Weiteren die Verwendung eines Protonen-translozierenden Retinalproteins und/oder einer photochrome Zusammensetzung als Sicherheitsfarbstoff bereit. In einer ersten Ausführungsform wird das Retinalprotein und/oder die photochrome Zusammensetzung auf ein sicherungsbedürftiges Dokument oder einen sicherungsbedürftigen Gegenstand aufgetragen. In einer weiteren Ausführungsform kann die auf das sicherungsbedürftige Dokument oder den sicherungsbedürftigen Gegenstand aufgetragene erfindungsgemäße photochrome Zusammensetzung (bzw. das Retinalprotein) auf dem Dokument oder Gegenstand fixiert werden.

Dabei kann die Fixierung gemäß der vorliegenden Erfindung durch physikalischen Einschluß oder kovalente Kopplung an das Dokument bewirkt werden. Erfindungsgemäß ist das sicherungsbedürftige Dokument beispielsweise ein Sicherheitspapier, ein Ausweis oder eine Banknote. Als Dokument kann jedoch erfindungsgemäß auch jedes weitere Dokument gelten, für das ein Sicherungsbedarf besteht.

Schließlich stellt die vorliegende Erfindung ein Verfahren zum Herstellen von Dokumenten mit Sicherheitsmerkmal bereit, das dadurch gekennzeichnet ist, dass vor, während oder nach der Herstellung eines Dokumentes in üblicher Weise ein erfindungsgemäßes Protonen-translozierendes Retinalprotein oder eine erfindungsgemäße photochrome Zusammensetzung aufgetragen und gegebenenfalls fixiert wird.

Die folgenden Figuren und Beispiele erläutern die Erfindung.

Figur 1 zeigt die Absorptionsspektren verschiedener erfindungsgemäßer Protonentranslozierender Retinalproteine im Vergleich zu dem Spektrum des Wildtyps bzw. von Muteinen mit nur einer Mutation. Die durchgezogenen Linien zeigen jeweils das Absorptionsspektrum im Hell-adaptierten Zustand, die gepunkteten Linien im Dunkel-adaptierten Zustand. Im Einzelnen zeigen:
- Figur 1A: Wildtyp *Halobacterium salinarum* mit der Aminosäuresequenz von SEQ ID No.1. Das Absorptionsmaximum im Hell-adaptierten Zustand liegt bei 568 nm, im Dunkel-adaptierten Zustand bei 560 nm.
- Figur 1B: Absorptionsspektrum der Typ 1-Mutante D96N. Das Absorptionsmaximum verschiebt sich von 569 nm im Hell-adaptierten Zustand nach 560 nm im Dunkel-adaptierten Zustand.
- Figur 1C: Typ 2-Mutante V49A. Das Absorptionsmaximum liegt im Hell-adaptierten wie im Dunkel-adaptierten Zustand bei 549 nm.
- Figur 1D: Erfindungsgemäßes Retinalprotein mit der Doppelmutation V49A-D96N. Das Absorptionsmaximum von 559 nm im Hell-adaptierten Zustand wird um 2 nm nach 557 nm im Dunkel-adaptierten Zustand verschoben.
- Figur 1 E: Typ 1-Mutation L93A. Das Absorptionsmaximum liegt im Hell-adaptierten wie im Dunkel-adaptierten Zustand bei 541 nm.
- Figur 1 F: Erfindungsgemäßes Retinalprotein mit der Doppelmutation L93A-D96N. Das Absorptionsmaximum liegt im Hell-adaptierten wie im Dunkel-adaptierten Zustand bei 544 nm. Diese Doppelmutante erreicht im Dunkel-adaptierten Zustand einen all*-trans* Anteil von mehr als 80%.
- Figur 1G: Typ 2-Mutation M145F. Das Absorptionsmaximum liegt im Hell-adaptierten Zustand bei 559 nm, im Dunkel-adaptierten Zustand bei 558 nm.
- Figur 1H: Erfindungsgemäßes Retinalprotein mit der Doppelmutation D96N-M145F. Diese Doppelmutante erreicht im Dunkel-adaptierten Zustand einen all*-trans* Anteil von 66%; das Absorptionsmaximum liegt für das Hell-adaptierte wie für das Dunkel-adaptierte Retinalprotein bei 560 nm.

### Beispiel 1

### Herstellung einer photochromen Zusammensetzung

Für die Herstellung einer photochromen Zusammensetzung werden zunächst die einzelnen Protonen-translozierenden Retinalproteine entweder aus natürlichen Halobakterienpopulationen oder rekombinant durch Transformation von *Halobacterium salinarum* (Cline & Doolittle, J. Bact. 169, 1341-1344, 1987) nach stellenspezifischer Mutagenese des Bakteriorhodopsingenes (Dunn et al., Proc. Natl. Acad. Sc., USA 78, 6744-6748) gewonnen. Die Purpurmembran der transformierten Halobakterien wird mittels bekannter Verfahren isoliert und gereinigt (Oesterhelt & Stoeckenius, Meth. Enzym. 31, 667-678, 1974). Für die Gewinnung von Bakteriorhodopsin in großem Maßstab kann das in der deutschen Patentanmeldung 199 45 798.0 beschriebene Verfahren eingesetzt werden.

### Beispiel 2

### Fixierung der photochromen Zusammensetzung auf einer Oberfläche

Die erfindungsgemäße photochrome Zusammensetzung kann beispielsweise physikalisch in ein Matrixmaterial eingeschlossen werden. Konkret werden z.B. 10 mg Purpurmembran, enthaltend Bakteriorhodopsin-D96N/M145F in 4 ml einer UV-härtenden Farbe (IFS 3000, Firma Schmitt) gleichmäßig suspendiert und auf das sicherungsbedürftige Dokument aufgetragen. Nach UV-Belichtung (nach Angaben des Herstellers) des solcherart markierten Dokumentes befinden sich die Purpurmembranpartikel im ausgehärteten Kunststoff.

### Beispiel 3

### Applikation der photochromen Zusammensetzung mittels verschiedener Verfahren

### Siebdruck

Das Prinzip des Siebdrucks ist Durchdruck, ähnlich einer Schabloniertechnik. Die Druckform besteht aus einem Siebgewebe, welches mit einer farbundurchlässigen Sperrschicht versehen wird. Das Druckmotiv bleibt offen. Der Druck erfolgt durch das Abziehen des mit Farbe gefüllten Siebes mit einer Rakel. Die Farbe wird dabei auf das darunterliegende Substrat übertragen. Zur Herstellung einer Siebdruckfarbe wird in eine 7,2% PVA-Lösung (Mowiol Typ 56-98) 100 mg/ml Purpurmembran über Nacht eingerührt. Bei Übereinstimmung der rheologischen Eigenschaften mit einer Standardprobe kann die erhaltene Mischung mit einer herkömmlichen Siebdruckmaschine verdruckt werden.

### Offsetdruck

In 5 ml einer Farbe ohne Pigment (Fa. Schmitt, UFO1) werden bei 50 °C 1g Purpurmembran eingerührt. Die so erhaltene Mischung kann mittels gängiger Offset-Technik verdruckt werden.

### Beispiel 4

### Herstellen eines abriebbeständigen Sicherheitsmerkmals

Eine Abriebbeständigkeit der photochromen Zusammensetzung kann erzielt werden, in dem z.B. die mit Protonen-translozierendem Retinalprotein beschichteten Dokumente mittels eines Heißlaminiergerätes (GPM, Mylam 9) in einer Folientasche vom Typ GHQ-120TR bei einer Temperatur von 90 bis 140 °C einlaminiert werden.

### Beispiel 5

### Erhöhung der UV-Beständigkeit des Sicherheitsmerkmals

Um die UV-Beständigkeit des erfindungsgemäßen Sicherheitsmerkmals zu erhöhen, wird die photochrome Zusammensetzung mit einem UV-Absorber oder einem Derivat davon in einer Konzentration von 1 bis 30%, bevorzugt 3 bis 10% w/w versetzt. Bevorzugte UV-Absorber sind Benzophenon, Hydroxynaphthochinon, Phenylbenzoxazol, Zimtsäureester, Sulfonamid und Aminobenzoesäureester.

## Patentansprüche

1. Photochrome Zusammensetzung, enthaltend mindestens ein Protonen-translozierendes Retinalprotein, ausgewählt aus der Gruppe von:
(i) Muteinen eines natürlichen Protonen-translozierenden Retinalproteins aus halophilen Archaebakterien, die einen verlangsamten Photozyklus autweisen (Typ 1-Mutation) und deren all-trans-Retinal-Gehalt im Hell- und im Dunkel-adaptierten Zustand um nicht mehr als 10% voneinander abweicht (Typ 2-Mutation) und/oder
(ii) Homologen der Muteine (i) mit verlangsamten Photozyklus, deren all-*trans-*Retinal-Gehalt im Hell- und im Dunkel-adaptierten Zustand um nicht mehr als 10% voneinander abweicht,
weiter enthaltend ein oder mehrere Zusatzstoffe, ausgewählt aus Stabilisatoren, die Schaumbildung verringernden Zusatzstoffen und UV-Licht absorbierenden Zusatzstoffen.

2. Photochrome Zusammensetzung nach Anspruch 1, **dadurch gekennzeichnet, dass** das Protonen-translozierende Retinalprotein eine Retinalisomeren-Zusammensetzung im Hell- und im Dunkel-adaptierten Zustand von mindestens 60% all-trans-Retinal aufweist.

3. Photochrome Zusammensetzung nach Anspruch 1 oder 2, **dadurch gekennzeichnet, dass** das natürliche Protonen-translozierende Retinalprotein ein archaebakterielles Rhodopsin ist.

4. Photochrome Zusammensetzung nach einem der Ansprüche 1 bis 3, **dadurch gekennzeichnet, dass** das archaebakterielle Rhodopsin ein Rhodopsin aus Halobakterien ist.

5. Photochrome Zusammensetzung nach einem der Ansprüche 1 bis 4, **dadurch gekennzeichnet, dass** das archaebakterielle Rhodopsin Bakteriorhodopsin (SEQ ID No.1) aus *Halobacterium salinarum* ist.

6. Photochrome Zusammensetzung nach einem der Ansprüche 1 bis 5, **dadurch gekennzeichnet, dass** das Homolog eine Aminosauresequenz aufweist, die mit der Aminosäuresequenz SEQ ID No.1 mindestens 40% Identität aufweist.

7. Photochrome Zusammensetzung nach einem der Ansprüche 1 bis 6, **dadurch gekennzeichnet, dass** das Homolog eine Aminosäuresequenz aufweist, die im Bereich der C- und/oder F-Helix mindestens 60% Identität mit der Aminosäuresequenz SEQ ID No.1 aufweist.

8. Photochrome Zusammensetzung nach mindestens einem der Ansprüche 1 bis 7, **dadurch gekennzeichnet, dass** der Photozyklus der Muteine mit einer Typ 1-Mutation eine thermische Zykluszeit von mehr als 10 ms aufweist.

9. Photochrome Zusammensetzung nach mindestens einem der Ansprüche 1 bis 8, **dadurch gekennzeichnet, dass** die Typ 1-Mutation ein Aminosäureaustausch an einer oder mehreren der Aminosäurepositionen ist, die im natürlichen Protein am katalytischen Zyklus beteiligt sind.

10. Photochrome Zusammensetzung nach Anspruch 9, **dadurch gekennzeichnet, dass** die am katalytischen Zyklus beteiligten Aminosäuren aus der die Aminosäurereste D38, R82, D85, D96, D102, D104, E194 und/oder E204 umfassenden Gruppe ausgewählt sind.

11. Photochrome Zusammensetzung nach mindestens einem der Ansprüche 1 bis 10, **dadurch gekennzeichnet, dass** die Typ 2-Mutation ein Aminosäureaustausch an einer oder mehrerer der Aminosäurepositionen ist, die die Retinalbindungstasche bilden.

12. Photochrome Zusammensetzung nach Anspruch 11, **dadurch gekennzeichnet, dass** die die Retinalbindungstaschen bildenden Aminosäuren aus der die Aminosäurereste Val49, Ala53, L93, Met118, Gly122, S141 und Met145 umfassenden Gruppe ausgewählt sind.

13. Photochrome Zusammensetzung nach einem der Ansprüche 1 bis 12, **dadurch gekennzeichnet, dass** es ein Mutein des Bakteriorhodopsins aus *Halobacterium salinarum* (SEQ 1 D No.1) mit aus der folgenden Gruppe ausgewählten Mutationen:
V49A-D96N; L93A-D96N; M145F-D96N
oder ein Homolog eines solchen Muteins ist.

14. Photochrome Zusammensetzung nach mindestens einem der Ansprüche 1 bis 13, **dadurch gekennzeichnet, dass** das Retinalprotein in Form einer Purpurmembran vorliegt.

15. Photochrome Zusammensetzung nach Anspruch 14, **dadurch gekennzeichnet, dass** die Dichte der Purpurmembran zwischen 1,10 und 1,20 g/cm³ beträgt

16. Photochrome Zusammensetzung nach Anspruch 15, **dadurch gekennzeichnet, dass** die Dichte der Purpurmembran 1,175 bis 1,185 g/cm³ beträgt.

17. Photochrome Zusammensetzung nach einem der Ansprüche 1-16, **dadurch gekennzeichnet, dass** Glycerin, organische Polymere und/oder organische Lösungsmittel enthalten sind.

18. Verwendung eines Protonen-translozierendes Retinalproteins, ausgewählt aus der Gruppe von:
(i) Muteinen eines natürlichen Protonen-translozierenden Retinalproteins aus halophilen Archaebakterien, die einen verlangsamten Photozyklus autweisen (Typ 1-Mutation) und deren all-*trans*-Retinal-Gehalt im Hell- und im Dunkel-adaptierten Zustand um nicht mehr als 10% voneinander abweicht (Typ 2-Mutation) und/oder
(iii) Homologen der Muteine (i) mit verlangsamten Photozyklus, deren all-*trans-*Retinal-Gehalt im Hell- und im Dunkel-adaptierten Zustand um nicht mehr als 10% voneinander abweicht,
und/oder einer photochromen Zusammensetzung nach einem der Ansprüche 1-17 zum Herstellen von optischen Filmen.

19. Verwendung nach Anspruch 18, **dadurch gekennzeichnet, dass** der optische Film für die optische Aufzeichnung geeignet ist.

20. Verwendung nach Anspruch 18, **dadurch gekennzeichnet, dass** der optische Film für die Interferometrie geeignet ist.

21. Verwendung nach Anspruch 18, **dadurch gekennzeichnet, dass** der optische Film für die holographische Mustererkennung geeignet ist.

22. Verwendung nach Anspruch 18, **dadurch gekennzeichnet, dass** der optische Film als optischer Lichtmodulator geeignet ist.

23. Verwendung eines Protonen-translozierendes Retinalproteins, ausgewählt aus der Gruppe von:
(i) Muteinen eines natürlichen Protonen-translozierenden Retinalproteins aus halophilen Archaebakterien, die einen verlangsamten Photozyklus aufweisen (Typ 1-Mutation) und deren all-*trans*-Retinal-Gehalt im Hell- und im Dunkel-adaptierten Zustand um nicht mehr als 10% voneinander abweicht (Typ 2-Mutation) und/oder
(iv) Homologen der Muteine (i) mit verlangsamten Photozyklus, deren all-*trans-*Retinal-Gehalt im Hell- und im Dunkel-adaptierten Zustand um nicht mehr als 10% voneinander abweicht,
zum Herstellen volumenhafter Speicher zur optischen Datenspeicherung.

24. Verwendung eines Protonen-translozierendes Retinalproteins, ausgewählt aus der Gruppe von:
(i) Muteinen eines natürlichen Protonen-translozierenden Retinalproteins aus halophilen Archaebakterien, die einen verlangsamten Photozyklus aufweisen (Typ 1-Mutation) und deren all-*trans*-Retinal-Gehalt im Hell- und im Dunkel-adaptierten Zustand um nicht mehr als 10% voneinander abweicht (Typ 2-Mutation) und/oder
(ii) Homologen der Muteine (i) mit verlangsamten Photozyklus, deren all-*trans-*Retinal-Gehalt im Hell- und im Dunkel-adaptierten Zustand um nicht mehr als 10% voneinander abweicht,
oder einer photochromen Zusammensetzung nach mindestens einem der Ansprüche 1-20 als Sicherheitsfarbstoff.

25. Verwendung nach Anspruch 24, **dadurch gekennzeichnet, dass** das Protonen-translozierende Retinalprotein und/oder die photochrome Zusammensetzung auf ein sicherungsbedürftiges Dokument oder einen sicherungsbedürftigen Gegenstand aufgetragen wird.

26. Verwendung nach Anspruch 25, **dadurch gekennzeichnet, dass** das auf das sicherungsbedürftige Dokument oder den Gegenstand aufgetragene Protonen-translozierende Retinalprotein und/oder die photochrome Zusammensetzung auf dem Dokument oder den Gegenstand fixiert wird.

27. Verwendung nach Anspruch 26, **dadurch gekennzeichnet, dass** die Fixierung durch physikalischen Einschluß oder kovalente Kopplung an das Dokument oder den Gegenstand bewirkt ist.

28. Verwendung nach einem der Anspruche 25-27, **dadurch gekennzeichnet, dass** das sicherungsbedürftige Dokument ein Sicherheitspapier ist.

29. Verwendung nach einem der Ansprüche 25-27, **dadurch gekennzeichnet, dass** das sicherungsbedürftige Dokument ein Ausweis ist.

30. Verwendung nach einem der Ansprüche 25-27, **dadurch gekennzeichnet, dass** das sicherungsbedürftige Dokument eine Banknote ist.

31. Verwendung nach einem der Ansprüche 18-30, **dadurch gekennzeichnet, dass** das Protonen-translozierende Retinalprotein eine Retinalisomeren-Zusammensetzung im Hell- und im Dunkel-adaptierten Zustand von mindestens 60% all-trans-Retinal aufweist.

32. Verwendung nach einem der Ansprüche 18-31, **dadurch gekennzeichnet, dass** das natürliche Protonen-translozierende Retinalprotein ein archaebakterielles Rhodopsin ist.

33. Verwendung nach einem der Ansprüche 18-32, **dadurch gekennzeichnet, dass** das archaebakterielle Rhodopsin ein Rhodopsin aus Halobakterien ist.

34. Verwendung nach einem der Ansprüche 18-33, **dadurch gekennzeichnet, dass** das archaebakterielle Rhodopsin Bakteriorhodopsin (SEQ ID No.1) aus *Halobacterium salinarum* ist.

35. Verwendung nach einem der Ansprüche 18-34, **dadurch gekennzeichnet, dass** das Homolog eine Aminosäuresequenz aufweist, die mit der Aminosäuresequenz SEQ ID No.1 mindestens 40% Identität aufweist.

36. Verwendung nach einem der Ansprüche 18-35, **dadurch gekennzeichnet, dass** das Homolog eine Aminosäuresequenz aufweist, die im Bereich der C- und/oder F-Helix mindestens 60% Identität mit der Aminosäuresequenz SEQ ID No.1 aufweist.

37. Verwendung nach einem der Ansprüche 18-36, **dadurch gekennzeichnet, dass** der Photozyklus der Muteine mit einer Typ 1-Mutation eine thermische Zykluszeit von mehr als 10 ms aufweist.

38. Verwendung nach einem der Ansprüche 18-37, **dadurch gekennzeichnet, dass** die Typ 1-Mutation ein Aminosäureaustausch an einer oder mehreren der Aminosäurepositionen ist, die im natürlichen Protein am katalytischen Zyklus beteiligt sind.

39. Verwendung nach Anspruch 38, **dadurch gekennzeichnet, dass** die am katalytischen Zyklus beteiligten Aminosäuren aus der die Aminosäurereste D38, R82, D85, D96, D102, D104, E194 und/oder E204 umfassenden Gruppe ausgewählt sind.

40. Verwendung nach einem der Ansprüche 18-39, **dadurch gekennzeichnet, dass** die Typ 2-Mutation ein Aminosäureaustausch an einer oder mehrerer der Aminosäurepositionen ist, die die Retinalbindungstasche bilden.

41. Verwendung nach Anspruch 40, **dadurch gekennzeichnet, dass** die die Retinalbindungstaschen bildenden Aminosäuren aus der die Aminosäurereste Val49, Ala53, L93, Met118, Gly122, S141 und Met145 umfassenden Gruppe ausgewählt sind.

42. Verwendung nach einem der Ansprüche 18-41, **dadurch gekennzeichnet, dass** es ein Mutein des Bakteriorhodopsins aus *Hatobacterium salinarum* (SEQ 1 D No.1) mit aus der folgenden Gruppe ausgewählten Mutationen:
V49A-D96N; L93A-D96N; M145F-D96N
oder ein Homolog eines solchen Muteins ist.

43. Verwendung nach mindestens einem der Ansprüche 18-42, **dadurch gekennzeichnet, dass** das Retinalprotein in Form einer Purpurmembran vorliegt.

44. Verwendung nach Anspruch 43, **dadurch gekennzeichnet, dass** die Dichte der Purpurmembran zwischen 1,10 und 1,20 g/cm³ beträgt

45. Verwendung nach Anspruch 44, **dadurch gekennzeichnet, dass** die Dichte der Purpurmembran 1,175 bis 1,185 g/cm³ beträgt.

46. Verfahren zum Herstellen von Dokumenten mit Sicherheitsmerkmal, **dadurch gekennzeichnet, dass** vor, während oder nach der Herstellung eines Dokumentes in üblicher Weise ein Protonen-translozierendes Retinalprotein, ausgewählt aus der Gruppe von:
(i) Muteinen eines natürlichen Protonen-translozierenden Retinalproteins aus halophilen Archaebakterien, die einen verlangsamten Photozyklus autweisen (Typ 1-Mutation) und deren all-*trans*-Retinal-Gehalt im Hell- und im Dunkel-adaptierten Zustand um nicht mehr als 10% voneinander abweicht (Typ 2-Mutation) und/oder
(ii) Homologen der Muteine (i) mit verlangsamten Photozyklus, deren all-*trans-*Retinal-Gehalt im Hell- und im Dunkel-adaptierten Zustand um nicht mehr als 10% voneinander abweicht,
oder eine photochrome Zusammensetzung gemäß einem der Ansprüche 1-17 aufgetragen' und gegebenenfalls fixiert wird.

47. Verfahren nach Anspruch 46, **dadurch gekennzeichnet, dass** das Protonen-translozierende Retinalprotein eine Retinalisomeren-Zusammensetzung im Hell- und im Dunkel-adaptierten Zustand von mindestens 60% all-trans-Retinal aufweist.

48. Verfahren nach Anspruch 46 oder 47, **dadurch gekennzeichnet, dass** das natürliche Protonen-translozierende Retinalprotein ein archaebakterielles Rhodopsin ist.

49. Verfahren nach einem der Ansprüche 46-48, **dadurch gekennzeichnet, dass** das archaebakterielle Rhodopsin ein Rhodopsin aus Halobakterien ist.

50. Verfahren nach einem der Ansprüche 46-49, **dadurch gekennzeichnet, dass** das archaebakterielle Rhodopsin Bakteriorhodopsin (SEQ ID No.1) aus *Halobacterium salinarum* ist.

51. Verfahren nach einem der Ansprüche 46-50, **dadurch gekennzeichnet, dass** das Homolog eine Aminosauresequenz aufweist, die mit der Aminosäuresequenz SEQ ID No.1 mindestens 40% Identität aufweist.

52. Verfahren nach einem der Ansprüche 46-51, **dadurch gekennzeichnet, dass** das Homolog eine Aminosäuresequenz aufweist, die im Bereich der C- und/oder F-Helix mindestens 60% Identität mit der Aminosäuresequenz SEQ ID No.1 aufweist.

53. Verfahren nach einem der Ansprüche 46-52, **dadurch gekennzeichnet, dass** der Photozyklus der Muteine mit einer Typ 1-Mutation eine thermische Zykluszeit von mehr als 10 ms aufweist.

54. Verfahren nach einem der Ansprüche 46-53, **dadurch gekennzeichnet, dass** die Typ 1-Mutation ein Aminosäureaustausch an einer oder mehreren der Aminosäurepositionen ist, die im natürlichen Protein am katalytischen Zyklus beteiligt sind.

55. Verfahren nach Anspruch 54, **dadurch gekennzeichnet, dass** die am katalytischen Zyklus beteiligten Aminosäuren aus der die Aminosäurereste D38, R82, D85, D96, D102, D104, E194 und/oder E204 umfassenden Gruppe ausgewählt sind.

56. Verfahren nach einem der Ansprüche 46-55, **dadurch gekennzeichnet, dass** die Typ 2-Mutation ein Aminosäureaustausch an einer oder mehrerer der Aminosäurepositionen ist, die die Retinalbindungstasche bilden.

57. Verfahren nach Anspruch 56, **dadurch gekennzeichnet, dass** die die Retinalbindungstaschen bildenden Aminosäuren aus der die Aminosäurereste Val49, Ala53, L93, Met118, Gly122, S141 und Met145 umfassenden Gruppe ausgewählt sind.

58. Verfahren nach einem der Ansprüche 46-57, **dadurch gekennzeichnet, dass** es ein Mutein des Bakteriorhodopsins aus *Ha*/*obacterium salinarum* (SEQ 1 D No.1) mit aus der folgenden Gruppe ausgewählten Mutationen:
V49A-D96N; L93A-D96N; M145F-D96N
oder ein Homolog eines solchen Muteins ist.

59. Verfahren nach einem der Ansprüche 46-58, **dadurch gekennzeichnet, dass** das Retinalprotein in Form einer Purpurmembran vorliegt.

60. Verfahren nach Anspruch 59, **dadurch gekennzeichnet, dass** die Dichte der Purpurmembran zwischen 1,10 und 1,20 g/cm³ beträgt

61. Verfahren nach Anspruch 60, **dadurch gekennzeichnet, dass** die Dichte der Purpurmembran 1,175 bis 1,185 g/cm³ beträgt.

62. Photochrome Zusammensetzung gemäß irgend einem der Ansprüche 1- 17, wobei als weiterer Zusatzstoff Puffersubstanzen enthalten sind.

## Claims

1. Photochromic composition which comprises at least one proton-translocating retinal protein selected from the group comprising:
(i) muteins of a natural proton-translocating retinal protein derived from halophilic archaebacteria which exhibit a retarded photocycle (type 1 mutation) and whose all-*trans* retinal contents in the light-adapted and dark-adapted states do not differ from each other by more than 10% (type 2 mutation) and/or
(ii) homologs of the muteins (i) which possess a retarded photocycle and whose all-*trans* retinal contents in the light-adapted and dark-adapted states do not differ from each other by more than 10%,
and further comprises one or more additives which is/are selected from stabilizers, foam formation-diminishing additives and UV light-absorbing additives.

2. Photochromic composition according to Claim 1,
**characterized in that** the proton-translocating retinal protein exhibits a retinal isomer composition of at least 60% all-trans retinal in both the light-adapted and dark-adapted states.

3. Photochromic composition according to Claim 1 or 2, **characterized in that** the natural proton-translocating retinal protein is an archaebacterial rhodopsin.

4. Photochromic composition according to one of Claims 1 to 3, **characterized in that** the archaebacterial rhodopsin is a rhodopsin derived from halobacteria.

5. Photochromic composition according to one of Claims 1 to 4, **characterized in that** the archaebacterial rhodopsin is *Halobacterium salinarum* bacteriorhodopsin (SEQ ID No. 1).

6. Photochromic composition according to one of Claims 1 to 5, **characterized in that** the homolog exhibits an amino acid sequence which exhibits at least 40% identity with the amino acid sequence SEQ ID No. 1.

7. Photochromic composition according to one of Claims 1 to 6, **characterized in that** the homolog exhibits an amino acid sequence which, in the region of the C helix and/or F helix, exhibits at least 60% identity with the amino acid sequence SEQ ID No. 1.

8. Photochromic composition according to at least one of Claims 1 to 7, **characterized in that** the photocycle of the muteins containing a type 1 mutation exhibits a thermal cycle time of more than 10 ms.

9. Photochromic composition according to at least one of Claims 1 to 8, **characterized in that** the type 1 mutation is an amino acid substitution at one or more of the amino acid positions which, in the natural protein, are involved in the catalytic cycle.

10. Photochromic composition according to Claim 9,
**characterized in that** the amino acids which are involved in the catalytic cycle are selected from the group comprising the amino acid residues D38, R82, D85, D96, D102, D104, E194 and/or E204.

11. Photochromic composition according to at least one of Claims 1 to 10, **characterized in that** the type 2 mutation is an amino acid substitution at one or more of the amino acid positions which form the retinal-binding pocket.

12. Photochromic composition according to Claim 11, **characterized in that** the amino acids which form the retinal-binding pockets are selected from the group which comprises the amino acid residues Val49, Ala53, L93, Met118, Gly122, S141 and Met145.

13. Photochromic composition according to one of Claims 1 to 12, **characterized in that** it is a mutein of the *Halobacterium salinarum* bacteriorhodopsin (SEQ ID No. 1) containing mutations selected from the following group:
V49A-D96N; L93A-D96N; M145F-D96N
or a homolog of such a mutein.

14. Photochromic composition according to at least one of Claims 1 to 13, **characterized in that** the retinal protein is present in the form of a purple membrane.

15. Photochromic composition according to Claim 14, **characterized in that** the density of the purple membrane is between 1.10 and 1.20 g/cm³.

16. Photochromic composition according to Claim 15, **characterized in that** the density of the purple membrane is from 1.175 to 1.185 g/cm³.

17. Photochromic composition according to one of Claims 1-16, **characterized in that** it comprises glycerol, organic polymers and/or organic solvents.

18. Use of a proton-translocating retinal protein which is selected from the group comprising:
(i) muteins of a natural proton-translocating retinal protein derived from halophilic archaebacteria which exhibit a retarded photocycle (type 1 mutation) and whose all-*trans* retinal contents in the light-adapted and dark-adapted states do not differ from each other by more than 10% (type 2 mutation) and/or
(iii) homologs of the muteins (i) which possess a retarded photocycle and whose all-*trans* retinal contents in the light-adapted and dark-adapted states do not differ from each other by more than 10%,
and/or of a photochromic composition according to one of Claims 1-17 for producing optical films.

19. Use according to Claim 18, **characterized in that** the optical film is suitable for optical recording.

20. Use according to Claim 18, **characterized in that** the optical film is suitable for interferometry.

21. Use according to Claim 18, **characterized in that** the optical film is suitable for holographic pattern recognition.

22. Use according to Claim 18, **characterized in that** the optical film is suitable as an optical light modulator.

23. Use of a proton-translocating retinal protein which is selected from the group comprising:
(i) muteins of a natural proton-translocating retinal protein derived from halophilic archaebacteria which exhibit a retarded photocycle (type 1 mutation) and whose all-*trans* retinal contents in the light-adapted and dark-adapted states do not differ from each other by more than 10% (type 2 mutation) and/or
(iv) homologs of the muteins (i) which possess a retarded photocycle and whose *all-trans* retinal contents in the light-adapted and dark-adapted states do not differ from each other by more than 10%, for producing capacious stores for optical data storage.

24. Use of a proton-translocating retinal protein which is selected from the group comprising:
(i) muteins of a natural proton-translocating retinal protein derived from halophilic archaebacteria which exhibit a retarded photocycle (type 1 mutation) and whose all-*trans* retinal contents in the light-adapted and dark-adapted states do not differ from each other by more than 10% (type 2 mutation) and/or
(ii) homologs of the muteins (i) which possess a retarded photocycle and whose *all-trans* retinal contents in the light-adapted and dark-adapted states do not differ from each other by more than 10%,
or of a photochromic composition according to at least one of Claims 1-20 as a security dye.

25. Use according to Claim 24, **characterized in that** the proton-translocating retinal protein and/or the photochromic composition is/are applied to a document which requires security or to an article which requires security.

26. Use according to Claim 25, **characterized in that** the proton-translocating retinal protein and/or the photochromic composition which has/have been applied to the document or article which requires security is/are fixed on the document or the article.

27. Use according to Claim 26, **characterized in that** the fixing is effected by physical inclusion or covalent coupling to the document or the article.

28. Use according to one of Claims 25-27, **characterized in that** the document which requires security is a security paper.

29. Use according to one of Claims 25-27, **characterized in that** the document which requires security is a pass.

30. Use according to one of Claims 25-27, **characterized in that** the document which requires security is a banknote.

31. Use according to one of Claims 18-30, **characterized in that** the proton-translocating retinal protein exhibits a retinal isomer composition of at least 60% all-trans retinal in both the light-adapted and dark-adapted states.

32. Use according to one of Claims 18-31, **characterized in that** the natural proton-translocating retinal protein is an archaebacterial rhodopsin.

33. Use according to one of Claims 18-32, **characterized in that** the archaebacterial rhodopsin is a rhodopsin described from halobacteria.

34. Use according to one of Claims 18-33, **characterized in that** the archaebacterial rhodopsin is *Halobacterium salinarum* bacteriorhodopsin (SEQ ID No. 1).

35. Use according to one of Claims 18-34, **characterized in that** the homolog exhibits an amino acid sequence which exhibits at least 40% identity with the amino acid sequence SEQ ID No. 1.

36. Use according to one of Claims 18-35, **characterized in that** the homolog exhibits an amino acid sequence which, in the region of the C helix and/or F helix, exhibits at least 60% identity with the amino acid sequence SEQ ID No. 1.

37. Use according to one of Claims 18-36, **characterized in that** the photocycle of the muteins containing a type 1 mutation exhibits a thermal cycle time of more than 10 ms.

38. Use according to one of Claims 18-37, **characterized in that** the type 1 mutation is an amino acid substitution at one or more of the amino acid positions which, in the natural protein, are involved in the catalytic cycle.

39. Use according to Claim 38, **characterized in that** the amino acids which are involved in the catalytic cycle are selected from the group comprising the amino acid residues D38, R82, D85, D96, D102, D104, E194 and/or E204.

40. Use according to one of Claims 18-39, **characterized in that** the type 2 mutation is an amino acid substitution at one or more of the amino acid positions which form the retinal-binding pocket.

41. Use according to Claim 40, **characterized in that** the amino acids which form the retinal-binding pockets are selected from the group comprising the amino acid residues Val49, Ala53, L93, Met118, Gly122, S141 and Met145.

42. Use according to one of Claims 18-41, **characterized in that** it is a mutein of the *Halobacterium salinarum* bacteriorhodopsin (SEQ ID No. 1) containing mutations selected from the following group:
V49A-D96N; L93A-D96N; M145F-D96N
or a homolog of such a mutein.

43. Use according to at least one of Claims 18-42, **characterized in that** the retinal protein is present in the form of a purple membrane.

44. Use according to Claim 43, **characterized in that** the density of the purple membrane is between 1.10 and 1.20 g/cm³.

45. Use according to Claim 44, **characterized in that** the density of the purple membrane is from 1.175 to 1.185 g/cm³.

46. Process for producing documents possessing security features, **characterized in that**, before, during or after the production of a document in a customary manner, a proton-translocating retinal protein which is selected from the group comprising:
(i) muteins of a natural proton-translocating retinal protein derived from halophilic archaebacteria which exhibit a retarded photocycle (type 1 mutation) and whose all-*trans* retinal contents in the light-adapted and dark-adapted states do not differ from each other by more than 10% (type 2 mutation) and/or
(ii) homologs of the muteins (i) which possess a retarded photocycle and whose *all-trans* retinal contents in the light-adapted and dark-adapted states do not differ from each other by more than 10%,
or a photochromic composition according to one of Claims 1-17 is applied and, where appropriate, fixed to it.

47. Process according to Claim 46, **characterized in that** the proton-translocating retinal protein exhibits a retinal isomer composition of at least 60% all-trans retinal in both the light-adapted and dark-adapted states.

48. Process according to Claim 46 or 47, **characterized in that** the natural proton-translocating retinal protein is an archaebacterial rhodopsin.

49. Process according to one of Claims 46-48, **characterized in that** the archaebacterial rhodopsin is a rhodopsin derived from halobacteria.

50. Process according to one of Claims 46-49, **characterized in that** the archaebacterial rhodopsin is *Halobacterium salinarum* bacteriorhodopsin (SEQ ID No. 1).

51. Process according to one of Claims 46-50, **characterized in that** the homolog exhibits an amino acid sequence which exhibits at least 40% identity with the amino acid sequence SEQ ID No. 1.

52. Process according to one of Claims 46-51, **characterized in that** the homolog exhibits an amino acid sequence which, in the region of the C helix and/or F helix, exhibits at least 60% identity with the amino acid sequence SEQ ID No. 1.

53. Process according to one of Claims 46-52, **characterized in that** the photocycle of the muteins containing a type 1 mutation exhibits a thermal cycle time of more than 10 ms.

54. Process according to one of Claims 46-53, **characterized in that** the type 1 mutation is an amino acid substitution at one or more of the amino acid positions which, in the natural protein, are involved in the catalytic cycle.

55. Process according to Claim 54, **characterized in that** the amino acids which are involved in the catalytic cycle are selected from the group comprising the amino acid residues D38, R82, D85, D96, D102, D104, E194 and/or E204.

56. Process according to one of Claims 46-55, **characterized in that** the type 2 mutation is an amino acid substitution at one or more of the amino acid positions which form the retinal-binding pocket.

57. Process according to Claim 56, **characterized in that** the amino acids which form the retinal-binding pockets are selected from the group comprising the amino acid residues Va149, Ala53, L93, Met118, Gly122, S141 and Met145.

58. Process according to one of Claims 46-57, **characterized in that** it is a mutein of the *Halobacterium salinarum* bacteriorhodopsin (SEQ ID No. 1) containing mutations selected from the following group:
V49A-D96N; L93A-D96N; M145F-D96N
or a homolog of such a mutein.

59. Process according to one of Claims 46-58, **characterized in that** the retinal protein is present in the form of a purple membrane.

60. Process according to Claim 59, **characterized in that** the density of the purple membrane is between 1.10 and 1.20 g/cm³.

61. Process according to Claim 60, **characterized in that** the density of the purple membrane is from 1.175 to 1.185 g/cm³.

62. Photochromic composition according to any one of Claims 1-17, wherein buffering substances are present as further additive.

## Revendications

1. Composition photochrome, contenant au moins une protéine de rétinal à translocation de protons, choisie dans le groupe :
(i) des mutéines d'une protéine naturelle du rétinal à translocation de protons provenant d'archéobactéries halophiles, qui présentent un photocycle ralenti (mutation de type 1) et dont les teneurs en tout-trans-rétinal dans l'état adapté à la clarté et dans l'état adapté à l'obscurité ne s'écartent pas l'une de l'autre de plus de 10 % (mutation de type 2) et/ou
(ii) des homologues des mutéines (i) ayant un photocycle ralenti, dont les teneurs en tout-trans-rétinal dans l'état adapté à la clarté et dans l'état adapté à l'obscurité ne s'écartent pas l'une de l'autre de plus de 10.%,
et contenant en outre un ou plusieurs additifs choisis parmi les stabilisants, les additifs diminuant la formation de mousse et les additifs absorbant la lumière UV.

2. Composition photochrome selon la revendication 1, **caractérisée en ce que** la protéine de rétinal à translocation de protons présente une composition d'isomères de rétinal, dans l'état adapté à la clarté et dans l'état adapté à l'obscurité, constituée d'au moins 60 % de tout-trans-rétinal.

3. Composition photochrome selon la revendication 1
ou 2, **caractérisée en ce que** la protéine naturelle de rétinal à translocation de protons est une rhodopsine archéobactérienne.

4. Composition photochrome selon l'une des revendications 1 à 3, **caractérisée en ce que** la rhodopsine archéobactérienne est une rhodopsine de halobactéries.

5. Composition photochrome selon l'une des revendications 1 à 4, **caractérisée en ce que** la rhodopsine archéobactérienne est la bactériorhodopsine (SEQ ID N°1) de *Halobacterium salinarum.*

6. Composition photochrome selon l'une des revendications 1 à 5, **caractérisée en ce que** l'homologue présente une séquence d'acides aminés qui présente une identité d'au moins 40 % avec la séquence d'acides aminés SEQ ID N°1.

7. Composition photochrome selon l'une des revendications 1 à 6, **caractérisée en ce que** l'homologue présente une séquence d'acides aminés qui dans le domaine de l'hélice C et/ou de l'hélice F présente une identité d'au moins 60 % avec la .séquence d'acides aminés SEQ ID N°1.

8. Composition photochrome selon au moins l'une des revendications 1 à 7, **caractérisée en ce que** le photocycle des mutéines ayant une mutation de type 1 présente une durée du cycle thermique supérieure à 10 ms.

9. Composition photochrome selon au moins l'une des revendications 1 à 8, **caractérisée en ce que** la mutation de type 1 est un échange d'acides aminés sur une ou plusieurs des positions d'acides aminés qui dans la protéine naturelle participent au cycle catalytique.

10. Composition photochrome selon la revendication 9, **caractérisée en ce que** les acides aminés participant au cycle catalytique sont choisis dans le groupe comprenant les résidus d'acides aminés D38, R82, D85, D96, D102, D104, E194 et/ou E204.

11. Composition photochrome selon au moins l'une des revendications 1 à 10, **caractérisée en ce que** la mutation de type 2 est un échange d'acides aminés sur une ou plusieurs des positions d'acides aminés qui forment la poche de liaison du rétinal.

12. Composition photochrome selon la revendication 11, **caractérisée en ce que** les acides aminés formant les poches de liaison du rétinal sont choisis dans le groupe comprenant les résidus d'acides aminés Va149, Ala53, L93, Met118, Gly122, S141 et Met145.

13. Composition photochrome selon l'une des revendications 1 à 12, **caractérisée en ce qu'**elle est une mutéine de la bactériorhodopsine de *Halobacterium salinarum* (SEQ ID N°1), avec les mutations choisies dans le groupe suivant :
V49A-D96N ; L93A-D96N ; M145F-D96N
ou un homologue d'une telle mutéine.

14. Composition photochrome selon au moins l'une des revendications 1 à 13, **caractérisée en ce que** la protéine de rétinal se présente sous forme d'une membrane pourpre.

15. Composition photochrome selon la revendication 14, **caractérisée en ce que** la masse volumique de la membrane pourpre est comprise entre 1,10 et 1,20 g/cm³.

16. Composition photochrome selon la revendication 15, **caractérisée en ce que** la masse volumique de la membrane pourpre est de 1,175 à 1,185 g/cm³.

17. Composition photochrome selon l'une des revendications 1 à 16, **caractérisée en ce qu'**elle contient du glycérol, des polymères organiques et/ou des solvants organiques.

18. Utilisation d'une protéine de rétinal à translocation de protons, choisie dans le groupe
(i) des mutéines d'une protéine naturelle du rétinal à translocation de protons provenant d'archéobactéries halophiles, qui présentent un photocycle ralenti (mutation de type 1) et dont les teneurs en tout-trans-rétinal dans l'état adapté à la clarté et dans l'état adapté à l'obscurité ne s'écartent pas l'une de l'autre de plus de 10 % (mutation de type 2) et/ou
(ii) des homologues des mutéines (i) ayant un photocycle ralenti, dont les teneurs en tout-trans-rétinal dans l'état adapté à la clarté et dans l'état adapté à l'obscurité ne s'écartent pas
l'une de l'autre de plus de 10 %, et/ou d'une composition photochrome selon l'une quelconque des revendications 1 à 17, pour fabriquer des films optiques.

19. Utilisation selon la revendication 18,
**caractérisée en ce que** le film optique convient à l'enregistrement optique.

20. Utilisation selon la revendication 18,
**caractérisée en ce que** le film optique convient à l'interférométrie.

21. Utilisation selon la revendication 18,
**caractérisée en ce que** le film optique convient à la reconnaissance holographique d'objets.

22. Utilisation selon la revendication 18,
**caractérisée en ce que** le film optique convient en tant que modulateur optique.

23. Utilisation d'une protéine de rétinal à translocation de protons, choisie dans le groupe :
(i) des mutéines d'une protéine naturelle du rétinal à translocation de protons provenant d'archéobactéries halophiles, qui présentent un photocycle ralenti (mutation de type 1) et dont les teneurs en tout-trans-rétinal dans l'état adapté à la clarté et dans l'état adapté à l'obscurité ne s'écartent pas l'une de l'autre de plus de 10 % (mutation de type 2) et/ou
(ii) des homologues des mutéines (i) ayant un photocycle ralenti, dont les teneurs en tout-trans-rétinal dans l'état adapté à la clarté et dans l'état adapté à l'obscurité ne s'écartent pas l'une de l'autre de plus de 10 %,
pour fabriquer des stockages en volume destinés à un stockage optique de données.

24. Utilisation d'une protéine de rétinal à translocation de protons, choisie dans le groupe :
(i) des mutéines d'une protéine naturelle du rétinal à translocation de protons provenant d'archéobactéries halophiles, qui présentent un photocycle ralenti (mutation de type 1) et dont les teneurs en tout-trans-rétinal dans l'état adapté à la clarté et dans l'état adapté à l'obscurité ne s'écartent pas l'une de l'autre de plus de 10 % (mutation de type 2) et/ou
(ii) des homologues des mutéines (i) ayant un photocycle ralenti, dont les teneurs en tout-trans-rétinal dans l'état adapté à la clarté et dans l'état adapté à l'obscurité ne s'écartent pas l'une de l'autre de plus de 10 %,
ou d'une composition photochrome selon au moins l'une des revendications 1 à 20, en tant que colorant de sécurité.

25. Utilisation selon la revendication 24,
**caractérisée en ce que** la protéine de rétinal à translocation de protons et/ou la composition photochrome sont appliquées sur un document exigeant une protection, ou un objet exigeant une protection.

26. Utilisation selon la revendication 25,
**caractérisée en ce que** la protéine de rétinal à translocation de protons et/ou la composition photochrome appliquées sur le document exigeant une protection ou sur l'objet, sont fixées sur le document
ou l'objet.

27. Utilisation selon la revendication 26,
**caractérisée en ce que** la fixation est réalisée par inclusion physique ou couplage covalent au document ou à l'objet.

28. Utilisation selon l'une des revendications 25 à 27, **caractérisée en ce que** le document exigeant une protection est un papier de sécurité.

29. Utilisation selon l'une des revendications 25 à 27, **caractérisée en ce que** le document exigeant une protection est une pièce d'identité.

30. Utilisation selon l'une des revendications 25 à 27, **caractérisée en ce que** le document exigeant une protection est un billet de banque.

31. Utilisation selon l'une des revendications 18 à 30, **caractérisée en ce que** la protéine de rétinal à translocation de protons présente une composition d'isomères de rétinal, dans l'état adapté à la clarté et dans l'état adapté à l'obscurité, constituée d'au moins 60 % de tout-trans-rétinal.

32. Utilisation selon l'une des revendications 18 à 31, **caractérisée en ce que** la protéine naturelle de rétinal à translocation de protons est une rhodopsine archéobactérienne.

33. Utilisation selon l'une des revendications 18 à 32, **caractérisée en ce que** la rhodopsine archéobactérienne est une rhodopsine de halobactéries.

34. Utilisation selon l'une des revendications 18 à 33, **caractérisée en ce que** la rhodopsine archéobactérienne est la bactériorhodopsine (SEQ ID N°1) de *Halobacterium salinarum.*

35. Utilisation selon l'une des revendications 18 à 34, **caractérisée en ce que** l'homologue présente une séquence d'acides aminés qui présente une identité d'au moins 40 % avec la séquence d'acides aminés SEQ ID N°1.

36. Utilisation selon l'une des revendications 18 à 35, **caractérisée en ce que** l'homologue présente une séquence d'acides aminés qui dans le domaine de l'hélice C et/ou de l'hélice F présente une identité d'au moins 60 % avec la séquence d'acides aminés SEQ ID N°1.

37. Utilisation selon l'une des revendications 18 à 36, **caractérisée en ce que** le photocycle des mutéines ayant une mutation de type 1 présente une durée du cycle thermique supérieure à 10 ms.

38. Utilisation selon l'une des revendications 18 à 37, **caractérisée en ce que** la mutation de type 1 est un échange d'acides aminés sur une ou plusieurs des positions d'acides aminés qui dans la protéine naturelle participent au cycle catalytique.

39. Utilisation selon la revendication 38,
**caractérisée en ce que** les acides aminés participant au cycle catalytique sont choisis dans le groupe comprenant les résidus d'acides aminés D38, R82, D85, D96, D102, D104, E194 et/ou E204.

40. Utilisation selon l'une des revendications 18 à 39, **caractérisée en ce que** la mutation de type 2 est un échange d'acides aminés sur une ou plusieurs des positions d'acides aminés qui forment la poche de liaison du rétinal.

41. Utilisation selon la revendication 40,
**caractérisée en ce que** les acides aminés formant les poches de liaison du rétinal sont choisis dans le groupe comprenant les résidus d'acides aminés Val49, Ala53, L93, Met118, Gly122, S141 et Met145.

42. Utilisation selon l'une des revendications 18 à 41, **caractérisée en ce qu'**elle est une mutéine de la bactériorhodopsine de *Halobacterium salinarum* (SEQ ID N°1), avec les mutations choisies dans le groupe suivant :
V49A-D96N ; L93A-D96N ; M145F-D96N
ou un homologue d'une telle mutéine.

43. Utilisation selon au moins l'une des revendications 18 à 42, **caractérisée en ce que** la protéine de rétinal se présente sous forme d'une membrane pourpre.

44. Utilisation selon la revendication 43,
**caractérisée en ce que** la masse volumique de la membrane pourpre est comprise entre 1,10 et 1,20 g/cm³.

45. Utilisation selon la revendication 44,
**caractérisée en ce que** la masse volumique de la membrane pourpre est de 1,175 à 1,185 g/cm³.

46. Procédé de fabrication de documents ayant une caractéristique de sécurité, **caractérisé en ce qu'**avant, pendant ou après la fabrication d'un document d'une manière usuelle, on applique une protéine de rétinal à translocation de protons choisie dans le groupe
(i) des mutéines d'une protéine naturelle du rétinal à translocation de protons provenant d'archéobactéries halophiles, qui présentent un photocycle ralenti (mutation de type 1) et dont les teneurs en tout-trans-rétinal dans l'état adapté à la clarté et dans l'état adapté à l'obscurité ne s'écartent pas l'une de l'autre de plus de 10 % (mutation de type 2) et/ou
(ii) des homologues des mutéines (i) ayant un photocycle ralenti, dont les teneurs en tout-trans-rétinal dans l'état adapté à la clarté et dans l'état adapté à l'obscurité ne s'écartent pas l'une de l'autre de plus de 10 %,
ou une composition photochrome selon l'une des revendications 1 à 17, et éventuellement on la fixe.

47. Procédé selon la revendication 46, **caractérisé en ce que** la protéine de rétinal à translocation de protons présente une composition d'isomères de rétinal, dans l'état adapté à la clarté et dans l'état adapté à l'obscurité, constituée d'au moins 60 % de tout-trans-rétinal.

48. Procédé selon la revendication 46 ou 47,
**caractérisé en ce que** la protéine naturelle de rétinal à translocation de protons est une rhodopsine archéobactérienne.

49. Procédé selon l'une des revendications 46 à 48, **caractérisé en ce que** la rhodopsine archéobactérienne est une rhodopsine de halobactéries.

50. Procédé selon l'une des revendications 46 à 49, **caractérisé en ce que** la rhodopsine archéobactérienne est la bactériorhodopsine (SEQ ID N°1) de *Halobacterium salinarum.*

51. Procédé selon l'une des revendications 46 à 50, **caractérisé en ce que** l'homologue présente une séquence d'acides aminés qui présente une identité d'au moins 40 % avec la séquence d'acides aminés SEQ ID N°1.

52. Procédé selon l'une des revendications 46 à 51, **caractérisé en ce que** l'homologue présente une séquence d'acides aminés qui dans le domaine de l'hélice C et/ou de l'hélice F présente une identité d'au moins 60 % avec la séquence d'acides aminés SEQ ID N°1.

53. Procédé selon l'une des revendications 46 à 52, **caractérisé en ce que** le photocycle des mutéines ayant une mutation de type 1 présente une durée du cycle thermique supérieure à 10 ms.

54. Procédé selon l'une des revendications 46 à 53, **caractérisé en ce que** la mutation de type 1 est un échange d'acides aminés sur une ou plusieurs des positions d'acides aminés qui dans la protéine naturelle participent au cycle catalytique.

55. Procédé selon la revendication 54, **caractérisé en ce que** les acides aminés participant au cycle catalytique sont choisis dans le groupe comprenant les résidus d'acides aminés D38, R82, D85, D96, D102, D104, E194 et/ou E204.

56. Procédé selon l'une des revendications 46 à 55, **caractérisé en ce que** la mutation de type 2 est un échange d'acides aminés sur une ou plusieurs des positions d'acides aminés qui forment la poche de liaison du rétinal.

57. Procédé selon la revendication 56, **caractérisé en ce que** les acides aminés formant les poches de liaison du rétinal sont choisis dans le groupe comprenant les résidus d'acides aminés Val49, Ala53, L93, Met118, Gly122, S141 et Met145.

58. Procédé selon l'une des revendications 46 à 57, **caractérisé en ce qu'**elle est une mutéine de la bactériorhodopsine de *Halobacterium salinarum* (SEQ ID N°1), avec les mutations choisies dans le groupe suivant :
V49A-D96N ; L93A-D96N ; M145F-D96N
ou un homologue d'une telle mutéine.

59. Procédé selon l'une des revendications 46 à 58, **caractérisé en ce que** la protéine de rétinal se présente sous forme d'une membrane pourpre.

60. Procédé selon la revendication 59, **caractérisé en ce que** la masse volumique de la membrane pourpre est comprise entre 1,10 et 1,20 g/cm³.

61. Procédé selon la revendication 60, **caractérisé en ce que** la masse volumique de la membrane pourpre est de 1,175 à 1,185 g/cm³.

62. Composition photochrome selon l'une quelconque des revendications 1 à 17, des substances tampons étant présentes en tant qu'additifs supplémentaires.


### SEQUENZPROTOKOLL

<110> Max-Planck-Gesellschaft
<120> Photochrome Zusammensetzung
<130> p31928
<140>
   <141>
<150> 1
<170> Patent In Ver. 2.1
<210> 1
   <211> 248
   <212> PRT
   <213> Malobacterium salinarum
<400> 1
   Glu Ala Gln Ile Thr Gly Arg Pro Glu Trp Ile Trp Leu Ala Leu Gly
     1               5                  10                  15
   Thr Ala Leu Met Gly Leu Gly Thr Leu Tyr Phe Leu Val Lys Gly Met
                20                  25                  30
   Gly Val Ser Asp Pro Asp Ala Lys Lys Phe Tyr Ala Ile Thr Thr Leu
            35                  40                  45
   Val Pro Ala Ile Ala Phe Thr Met Tyr Leu Ser Met Leu Leu Gly Tyr
        50                  55                  60
   Gly Leu Thr Met Val Pro Phe Gly Gly Glu Gln Asn Pro Ile Tyr Trp
    65                  70                  75                  80
   Ala Arg Tyr Ala Asp Trp Leu Phe Thr Thr Pro Leu Leu Leu Leu Asp
                    85                  90                  95
   Leu Ala Leu Leu Val Asp Ala Asp Gln Gly Thr Ile Leu Ala Leu Val
               100                 105                 110
   Gly Ala Asp Gly Ile Met Ile Gly Thr Gly Leu Val Gly Ala Leu Thr
           115                 120                 125
   Lys Val Tyr Ser Tyr Arg Phe Val Trp Trp Ala Ile Ser Thr Ala Ala
       130                 135                 140
   Met Leu Tyr Ile Leu Tyr Val Leu Phe Phe Gly Phe Thr Ser Lys Ala
   145                 150                 155                 160
   Glu Ser Met Arg Pro Glu Val Ala Ser Thr Phe Lys Val Leu Arg Asn
                   165                 170                 175
   Val Thr Val Val Leu Trp Ser Ala Tyr Pro Val Val Trp Leu Ile Gly
               180                 185                 190
   Ser Glu Gly Ala Gly Ile Val Pro Leu Asn Ile Glu Thr Leu Leu Phe
           195                 200                 205
   Met Val Leu Asp Val Ser Ala Lys Val Gly Phe Gly Leu Ile Leu Leu
       210                 215                 220
   Arg Ser Arg Ala Ile Phe Gly Glu Ala Glu Ala Pro Glu Pro Ser Ala
   225                 230                 235                 240
   Gly Asp Gly Ala Ala Ala Thr Ser
                   245